(19) Europäisches Patentamt

European Patent Office

Office européen des brevets

(11) **EP 3 946 040 B1**

(12) **FASCICULE DE BREVET EUROPEEN**

(45) Date de publication et mention
de la délivrance du brevet:
**06.11.2024 Bulletin 2024/45**

(51) Classification Internationale des Brevets (IPC):
**A61B 5/053** (2021.01)      **A61B 5/00** (2006.01)
**A61B 5/113** (2006.01)

(52) Classification Coopérative des Brevets (CPC):
**A61B 5/0535; A61B 5/1135; A61B 5/6805;**
A61B 2503/40; A61B 2503/42

(21) Numéro de dépôt: **20715899.9**

(22) Date de dépôt: **06.04.2020**

(86) Numéro de dépôt international:
**PCT/EP2020/059799**

(87) Numéro de publication internationale:
**WO 2020/201582 (08.10.2020 Gazette 2020/41)**

(54) **GILET DE PLETHYSMOGRAPHIE PAR INDUCTANCE POUR UN PETIT MAMMIFERE ET PROCEDE DE FABRICATION D'UN TEL GILET**

WESTE ZUR INDUKTIVEN PLETHYSMOGRAPHIE FÜR KLEINE SÄUGETIERE UND VERFAHREN ZUR HERSTELLUNG EINER SOLCHEN WESTE

INDUCTANCE PLETHYSMOGRAPHY VEST FOR A SMALL MAMMAL AND METHOD FOR PRODUCING SUCH A VEST

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **05.04.2019 FR 1903671**

(43) Date de publication de la demande:
**09.02.2022 Bulletin 2022/06**

(73) Titulaires:
• **Etisense**
**69008 Lyon (FR)**
• **Université Grenoble Alpes**
**38400 Saint-Martin-d'Hères (FR)**

(72) Inventeurs:
• **EYNARD, Charles**
**69003 Lyon (FR)**
• **FLENET, Timothé**
**69001 Lyon (FR)**
• **FONTECAVE-JALLON, Julie**
**38700 Corenc (FR)**
• **TANGUY, Stéphane**
**38330 Montbonnot-Saint-Martin (FR)**

(74) Mandataire: **Regimbeau**
**20, rue de Chazelles**
**75847 Paris Cedex 17 (FR)**

(56) Documents cités:
EP-A2- 1 404 219        EP-A2- 1 404 219
WO-A1-2017/037369        WO-A1-2017/037369
US-A- 5 543 012        US-A- 5 543 012
US-A1- 2006 258 914        US-A1- 2006 258 914
US-A1- 2018 317 814        US-A1- 2018 317 814

**Description**

**DOMAINE DE L'INVENTION**

L'invention concerne un gilet de pléthysmographie par inductance pour un petit mammifère et un procédé de fabrication d'un tel gilet.

**ETAT DE LA TECHNIQUE**

La pléthysmographie par inductance est un outil de mesure des variations de volume du tronc d'un mammifère (humain ou animal), pour des applications respiratoires et/ou cardiaques.

Le principe de cette mesure est d'enrouler autour du tronc du sujet au moins une spire inductive formée d'un ou plusieurs brins de fil électriquement conducteur. Une variation de volume du tronc, due notamment à l'activité respiratoire et/ou cardiaque, engendre une variation d'inductance de chaque spire, qui est mesurée par un capteur.

La fiabilité des mesures nécessite de s'assurer que la position de la (les) spire(s) par rapport au sujet soit constante au cours du temps. En particulier, il est nécessaire d'éviter tout glissement de chaque spire vis-à-vis du tronc du sujet. Par ailleurs, dans le cas d'une pluralité d'enregistrements successifs, il est nécessaire de positionner chaque spire au même emplacement sur le tronc du sujet pour assurer une reproductibilité des mesures.

Pour l'être humain, il est connu de coudre chaque spire sur une bande d'un textile élastique et d'enrouler ladite bande autour du tronc du sujet. De manière alternative, chaque spire ou bande peut être cousue sur un vêtement, tel qu'un T-shirt, destiné à être enfilé sur le tronc du sujet avec un léger serrage, ce qui facilite la mise en place du dispositif de pléthysmographie. On pourra se référer à cet égard au document US6341504.

Pour les animaux de taille moyenne (tels que chien, primate, porc), il est également connu d'utiliser de telles bandes, qui sont maintenues sur l'animal en étant intégrées à un vêtement par des boutons pression. Le document US7762953 décrit un tel dispositif.

Ces bandes ou vêtements nécessitent une confection précise afin de procurer un positionnement correct des spires et permettre une bonne reproductibilité des mesures au cours du temps et d'un dispositif à l'autre. La fixation des spires doit en outre ne pas conduire à une rigidification du gilet afin de ne pas empêcher la variation de section des spires et de minimiser la contrainte sur le tronc du sujet.

Pour l'homme, chez qui le dispositif doit être réutilisable, la couture est généralement réalisée manuellement, ce qui induit un coût de fabrication élevé ou par des techniques de tricotage de se limitant à la réalisation de bandes élastiques pouvant être par la suite intégrées dans un vêtement, comme décrit dans le document US6341504 susmentionné.

Les documents US 5,543,012 et US 2018/0317814 décrivent respectivement une bande et un gilet de pléthysmographie par inductance pour un être humain.

Cependant, pour les petits mammifères de laboratoire, une telle confection pose trois problèmes principaux. Par « petit mammifère », on entend dans le présent texte les mammifères habituellement utilisés en laboratoire et dont la masse est inférieure à 6 kg. Ces mammifères comprennent en particulier les rongeurs (souris, rats, cobayes, hamsters, gerbilles, furets) et les lagomorphes (lapins).

Premièrement, la taille des spires est tellement réduite par rapport à celle d'un humain ou d'un animal de taille moyenne qu'elle rend la couture manuelle malaisée. Deuxièmement, il est malaisé de tricoter et d'assembler des bandes élastiques de petite taille sur un tel dispositif sans le rigidifier. Troisièmement, le dispositif est destiné à être porté par des mammifères dont les comportements sont de nature à le dégrader (perforations, déchirements, arrachements, souillures). Son remplacement régulier étant inévitable, son coût doit donc être minimisé.

Par exemple, le document WO 2017/037369 décrit un dispositif de pléthysmographie par inductance pour la mesure non-invasive du débit aortique au niveau sous-diaphragmatique chez un petit mammifère de laboratoire.

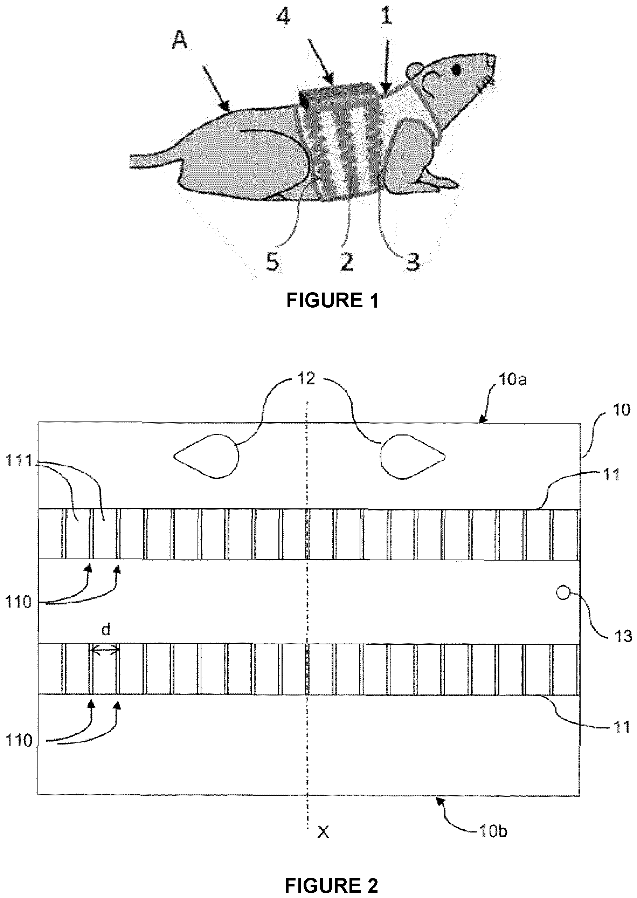

Comme illustré sur la figure 1, le petit mammifère A est équipé d'un gilet 1 de pléthysmographie du thorax par inductance (PTI) comportant deux spires extensibles électriquement conductrices 2, 3 solidaires d'un tube en textile élastique ajustable au tronc dudit mammifère. De manière optionnelle, le gilet 1 peut comprendre en outre une spire 5 supplémentaire agencée au niveau abdominal pour permettre une mesure de pléthysmographie respiratoire par inductance (PRI).

Les spires 2 et 3 sont connectées à un dispositif 4 d'acquisition qui permet d'enregistrer le signal de variation de section de chaque spire.

Les spires 2 et 3 sont conformées en vaguelettes pour accompagner les déformations des sections qu'elles entourent et sont positionnées autour de deux régions du thorax de l'animal.

Le positionnement et la configuration des spires (amplitude et période spatiale des vaguelettes) sont déterminés par un modèle physiologique fonctionnel sur lequel est basé le calcul du débit aortique sous-diaphragmatique et standardisés selon des critères morphologiques (gammes de poids, espèces concernées). Pour des petits mammifères, le diamètre des spires à l'état libre est typiquement compris entre 2 et 15 cm. Par ailleurs, l'espace entre les deux spires est typiquement compris entre 0,5 et 5 cm. En ce qui concerne l'amplitude et la période spatiale des vaguelettes des spires, elles sont respectivement de l'ordre de 0,5 à 5 cm et de 0,5 à 3 cm.

Le dispositif comprend en outre un processeur configuré pour calculer le débit aortique sous-diaphragmatique instantané du petit mammifère, à partir desdits signaux de variation de section de chaque spire et à partir d'un modèle fonctionnel du système cardio-respiratoire selon lequel les échanges de sang entre le thorax et le reste du corps dudit mammifère consistent en une sortie de sang par l'aorte abdominale au niveau sous-diaphragmatique et en une entrée de sang par la veine cave inférieure.

Le document EP 1 404 219 décrit un dispositif de pléthysmographie par inductance dans lequel une spire inductive est incorporée dans un tissu élastique par tricotage, couture, ou tressage.

Il n'existe pas à ce jour de procédé permettant de fabriquer le gilet avec une qualité suffisamment précise et un coût satisfaisant.

## EXPOSE DE L'INVENTION

Un but de l'invention est donc de concevoir un gilet de pléthysmographie par inductance pour petit mammifère de laboratoire qui présente une bonne reproductibilité de la position et de la taille des spires et qui soit peu onéreux à fabriquer.

A cet effet, l'invention propose un gilet de pléthysmographie par inductance pour un petit mammifère, c'est-à-dire un mammifère présentant une masse inférieure à 6 kg, comprenant :

- un tube en textile élastique configuré pour entourer le tronc dudit mammifère, ledit tube s'étendant selon un axe longitudinal adapté pour être parallèle à la colonne vertébrale dudit mammifère ;
- au moins une spire inductive formée d'un brin de fil électriquement conducteur agencé sous forme de vaguelettes selon la circonférence dudit tube ;

ledit gilet étant caractérisé en ce qu'il comprend au moins une lanière en textile élastique, agencée le long de la circonférence du tube, définissant une pluralité de passants longitudinaux, chaque vaguelette étant formée par le passage du brin de fil conducteur successivement dans chaque passant.

La circonférence du tube s'étend autour de l'axe longitudinal du tube. En d'autres termes, lorsque le tube est présenté à plat, sa circonférence est perpendiculaire à l'axe longitudinal.

Par ailleurs, la lanière en textile élastique est distincte du tube et assemblée sur celui-ci, par des moyens détaillés plus bas.

Ainsi, chaque passant se présente comme un anneau délimité d'une part par le tube et d'autre part par la lanière, s'étendant dans la direction longitudinale du tube, et présentant deux extrémités opposées dans ladite direction longitudinale, adapté pour passer un brin de fil conducteur à l'intérieur dudit anneau afin, de le maintenir entre lesdites extrémités, à la manière du passant d'une ceinture.

Les passants constituent avantageusement le seul moyen de maintien de la forme des vaguelettes, et permettent notamment de s'affranchir de tout autre moyen de fixation du brin de fil conducteur sur le tube, tel que la couture, le tricotage, le collage, etc.

En particulier, les passants procurent une liaison glissante entre le brin de fil conducteur et le tube, contrairement aux techniques précédemment citées qui réalisent une liaison rigide entre le brin de fil conducteur et le tube. Cette liaison présente plusieurs avantages. D'une part, elle permet de minimiser la rigidification du gilet grâce à la possibilité pour le brin de fil de coulisser à l'intérieur des passants lors d'une déformation du tube. D'autre part, elle permet un remplacement aisé du brin de fil conducteur, par exemple en cas d'endommagement de celui-ci.

De manière particulièrement avantageuse, chaque passant est délimité par deux lignes longitudinales d'assemblage de la lanière sur le tube.

De préférence, la distance entre deux lignes d'assemblage adjacentes est constante.

Selon un mode de réalisation, le tube comprend en outre deux orifices adaptés pour le passage des pattes avant du mammifère, chaque orifice présentant une forme allongée selon la circonférence du tube.

De manière avantageuse, le tube comprend en outre un renfort formé par un rabat du tube du côté des pattes avant du mammifère, lesdits orifices s'étendant au travers dudit rabat.

Selon un mode de réalisation, le gilet comprend en outre un logement pour un dispositif d'acquisition connecté à chaque spire inductive, ledit logement étant formé d'un seul tenant avec le tube ou solidarisé directement au tube.

Selon un autre mode de réalisation, le gilet comprend en outre un harnais comprenant un logement pour un dispositif d'acquisition connecté à chaque spire inductive et une sangle adaptée pour maintenir le tube autour de l'abdomen du mammifère.

Ladite sangle peut être pourvue d'une pluralité de pièces agrippantes de type crochets et boucles.

Selon un mode de réalisation, chaque brin de fil électriquement conducteur passe au moins deux fois dans chaque passant selon la circonférence du tube, de sorte à former un ensemble de spires constituant une bobine.

Un autre objet de l'invention concerne un procédé de fabrication d'un tel gilet. Ledit procédé comprend :

- la fourniture d'une bande en textile élastique présentant un axe longitudinal ;
- l'assemblage sur la bande d'au moins une lanière en textile élastique perpendiculaire à l'axe longitudinal, selon une pluralité de lignes parallèles audit axe longitudinal, de sorte à définir une pluralité de passants entre deux lignes adjacentes de ladite lanière ;

- la fermeture de la bande selon une ligne parallèle à l'axe longitudinal de sorte à former un tube ;

- la formation d'au moins une spire inductive selon la circonférence du tube en passant un brin de fil électriquement conducteur successivement dans chaque passant d'une lanière respective de sorte à former des vaguelettes.

L'assemblage de la lanière sur le tube peut être avantageusement réalisé par soudure par ultrason, couture et/ou collage.

Selon un mode de réalisation, le procédé comprend en outre le perçage dans la bande de deux orifices adaptés pour le passage des pattes avant du petit mammifère au moyen d'un poinçon.

Le procédé peut en outre comprendre, avant le perçage desdits orifices, l'assemblage d'un rabat de la bande sur une face du tube, les orifices étant percés au travers dudit rabat.

L'invention concerne également un procédé de fabrication d'un ensemble de gilets de pléthysmographie par inductance pour des petits mammifères de tailles différentes, la masse desdits mammifères étant comprise entre 20 et 6000 g, de préférence entre 100 et 6000 g, dans lequel on met en oeuvre le procédé tel que décrit ci-dessus pour chacun desdits gilets, la distance entre deux lignes d'assemblage adjacentes de chaque lanière étant identique pour l'ensemble des gilets.

**BREVE DESCRIPTION DES FIGURES**

D'autres caractéristiques et avantages de l'invention ressortiront de la description détaillée qui va suivre, en référence aux dessins annexés sur lesquels :

- la figure 1 est une vue schématique d'un rat portant un gilet de pléthysmographie par inductance tel que décrit dans le document WO 2017/037369 ;
- la figure 2 est une vue du patron du gilet (du côté de sa face intérieure) selon un mode de réalisation ;
- la figure 3 est une vue du patron de la figure 2, du côté de la face extérieure du gilet ;
- la figure 4 est une vue de la bande assemblée ;
- la figure 5 est une vue de la bande assemblée avec des spires inductives multiples ;
- la figure 6 est une vue du patron du harnais (du côté de sa face extérieure) selon un mode de réalisation ;
- la figure 7 est une vue du patron de la figure 6, du côté de la face intérieure du harnais ;
- la figure 8 est une vue du gilet assemblé après fermeture de la bande sur elle-même pour former le tube ;
- la figure 9 est une vue en perspective d'un gilet assemblé selon un autre mode de réalisation,
- la figure 10 est une vue du patron du gilet de la figure 9.

Les signes de référence identiques d'une figure à l'autre désignent des éléments identiques ou remplissant la même fonction.

**DESCRIPTION DETAILLEE DE MODES DE REALISATION**

Selon l'invention, le gilet de pléthysmographie par inductance pour un petit mammifère comprend un tube en textile élastique configuré pour entourer le tronc dudit mammifère.

Le tube comprend un axe longitudinal qui est sensiblement parallèle à la colonne vertébrale de l'animal.

Le tube peut présenter une fermeture longitudinale, par exemple agencée le long de la colonne vertébrale de l'animal, afin de faciliter la mise en place du tube sur le tronc. Une telle fermeture peut comprendre des éléments d'attache de type crochets et boucles (plus connus sous le nom VELCRO™), des boutons pression, une fermeture à glissière, etc.

De manière alternative, le tube est fermé sur lui-même de manière permanente, par soudure, collage ou couture par exemple ; il doit alors être enfilé sur le tronc de l'animal, l'élasticité du textile étant choisie pour permettre un tel enfilage avec ajustement au tronc de l'animal.

Le tube sert de support à au moins une spire inductive formée d'un brin de fil électriquement conducteur agencé sous forme de vaguelettes selon la circonférence dudit tube.

A cet effet, le gilet comprend au moins une lanière en textile élastique agencée sur la circonférence du tube et définissant une pluralité de passants longitudinaux, chaque vaguelette étant formée par le passage du brin de fil conducteur successivement dans chaque passant.

La lanière est assemblée sur le tube selon des lignes longitudinales, de sorte que deux lignes adjacentes définissent un passant. L'assemblage peut être réalisé par soudure, par couture, par collage, ou tout autre moyen adapté.

La soudure (notamment la soudure par ultrason) est particulièrement préférée dans la mesure où elle peut être réalisée à l'aide d'un dispositif d'encombrement réduit, du côté extérieur de la lanière (qui est facilement accessible) ; de plus, cette soudure peut être mise en oeuvre au moyen d'un gabarit servant à guider la soudure selon des lignes longitudinales et permettant de définir la largeur des passants.

Etant réalisé dans le sens longitudinal, cet assemblage n'affecte pas l'élasticité du tube et de chaque lanière dans le sens de la circonférence. Ainsi, il n'entrave pas la déformation de la (les) spire(s) au cours de l'activité de l'animal et ne perturbe pas les mesures pléthysmographiques. Même si l'assemblage est susceptible de rigidifier le tube dans le sens longitudinal, une telle rigidification n'est pas pénalisante pour les mesures.

La largeur des passants (distance entre deux lignes adjacentes) définit la période de chaque spire. Cette largeur est typiquement choisie pour ajuster le nombre de vaguelettes entre une valeur minimale (par exemple 10) permettant de garantir l'élasticité de chaque spire et une valeur maximale (par exemple 20) permettant de limiter le temps nécessaire à la mise en place du fil dans l'ensemble des passants. En pratique, une largeur de l'ordre de 4 à 40 mm est préférée. Le gabarit susmentionné permet d'assurer une période des spires constante sur toute la circonférence du tube.

De manière particulièrement avantageuse, cette largeur des passants peut même être constante entre des gilets de tailles différentes. En effet, pour des petits mammifères dont la masse varie entre 20 g et 6 kg, il est possible de définir un nombre limité de gilets de tailles différentes, chacune adaptée à une gamme donnée de petits mammifères. La conservation de la largeur des passants d'une taille à l'autre permet de simplifier la fabrication du gilet et de conserver la même période pour chaque spire.

Selon d'autres modes de réalisation, le gilet peut comprendre deux spires inductives ou davantage, agencés à différents emplacements du thorax et/ou de l'abdomen de l'animal. Dans ce cas, le gilet comprend autant de lanières que de spires, lesdites lanières étant agencées parallèlement les unes aux autres sur la longueur du tube, chaque lanière définissant une pluralité de passants longitudinaux dans lesquels chaque fil électriquement conducteur est agencé pour former des vaguelettes.

Selon une forme d'exécution avantageuse, chaque brin de fil électriquement conducteur passe au moins deux fois dans les passants d'une même lanière selon la circonférence du tube, de sorte à former un ensemble de spires constituant une bobine. Ceci permet d'augmenter la sensibilité des spires. L'auto-inductance L (en henry (H)) d'une bobine à air est en effet définie par la formule :

$$L = \frac{\mu_0 N^2 S}{l}$$

où $\mu_0$ est la constante magnétique, égale à $4\pi*10^{-7}$ $H.m^{-1}$, N est le nombre de spires, S est la section de la bobine en $m^2$, et l est la longueur de la bobine en m.

La valeur d'auto-inductance est multipliée par un facteur $N^2/l$ pour une même section entourée. Par rapport à une seule spire, une bobine constituée de N spires nécessite un fil N fois plus long. L'auto-inductance de chaque capteur ($L_N$) est ainsi théoriquement égale à l'auto-inductance d'une spire ($L_{N=1}$) multipliée par un facteur N.

De manière avantageuse, le gilet comprend deux orifices pour le passage des pattes avant de l'animal. Dans la mesure où la section des pattes n'est pas circulaire, lesdits orifices sont de préférence non circulaires. De préférence, lesdits orifices présentent une forme allongée (par exemple une forme de type goutte d'eau) dans le sens de la circonférence du tube. Ainsi, le gilet peut s'ajuster plus étroitement au tronc de l'animal dans la région des pattes avant. Cette forme est mieux adaptée à la morphologie des membres de l'animal ; elle prévient la gêne liée au port du gilet et assure un meilleur maintien dans le temps.

La partie avant du tube (située du côté des pattes avant) peut être rigidifiée par un renfort. De manière particulièrement avantageuse, ledit renfort peut être constitué par un rabat du tube du côté extérieur de celui-ci, assemblé sur la face extérieure du tube par l'un des procédés d'assemblage mentionnés plus haut. De préférence, ce renfort s'étend au moins jusqu'à l'arrière des pattes avant de l'animal ; ainsi, les orifices pour les pattes avant s'étendent au travers d'une double épaisseur de textile élastique, ce qui contribue à renforcer la tenue mécanique du tube dans cette région.

Selon un mode de réalisation, le gilet comprend en outre un harnais destiné à supporter un connecteur électriquement connecté au fil électriquement conducteur et/ou à augmenter le maintien du tube sur le tronc de l'animal.

Ce harnais comprend avantageusement une poche adaptée pour recevoir un connecteur adapté pour relier électriquement le(s) fil(s) électriquement conducteur(s) et la chaîne de mesure pléthysmographique. Ladite poche est destinée à être positionnée sur le dos de l'animal.

De manière alternative, la poche peut être formée directement sur la face extérieure du tube.

Le gilet décrit plus haut peut être fabriqué de manière simple tout en assurant une bonne précision et reproductibilité du positionnement des spires.

Le tube est formé à partir d'une bande en textile élastique qui est fermée sur elle-même selon une ligne parallèle à l'axe longitudinal du tube.

La fermeture peut être permanente, c'est-à-dire non démontable sans endommagement du gilet, par exemple par soudure de la bande sur elle-même.

De manière alternative, la fermeture peut être réversible, par exemple grâce à des pièces agrippantes de type crochets et boucles, des boutons, etc.

Avant d'être fermée sur elle-même, la bande est assemblée à au moins une lanière selon des lignes longitudinales qui délimitent les passants dans ladite lanière. Comme indiqué plus haut, cet assemblage peut être réalisé par soudure (notamment soudure par ultrason), collage, couture, etc. L'avantage de cette opération est qu'elle est réalisée à plat, un gabarit pouvant être utilisé pour garantir une distance constante entre lignes d'assemblage.

Avant ou après la fermeture du tube sur lui-même, un fil électriquement conducteur est enfilé successivement dans l'ensemble des passants d'une même lanière. Une fois le tube fermé, le fil forme ainsi une spire inductive. Pour faciliter la mise en place du fil dans les passants étroits, le fil est avantageusement enfilé dans le chas d'une aiguille et introduit dans le passant au moyen de ladite aiguille.

Chaque extrémité du fil est ensuite sertie dans une cosse et ladite cosse est insérée dans un connecteur qui est positionné sur le dos de l'animal dans une poche prévue à cet effet. Le connecteur est lui-même relié à la chaîne de mesure pléthysmographique.

Les figures 2 à 7 illustrent différents composants du gilet.

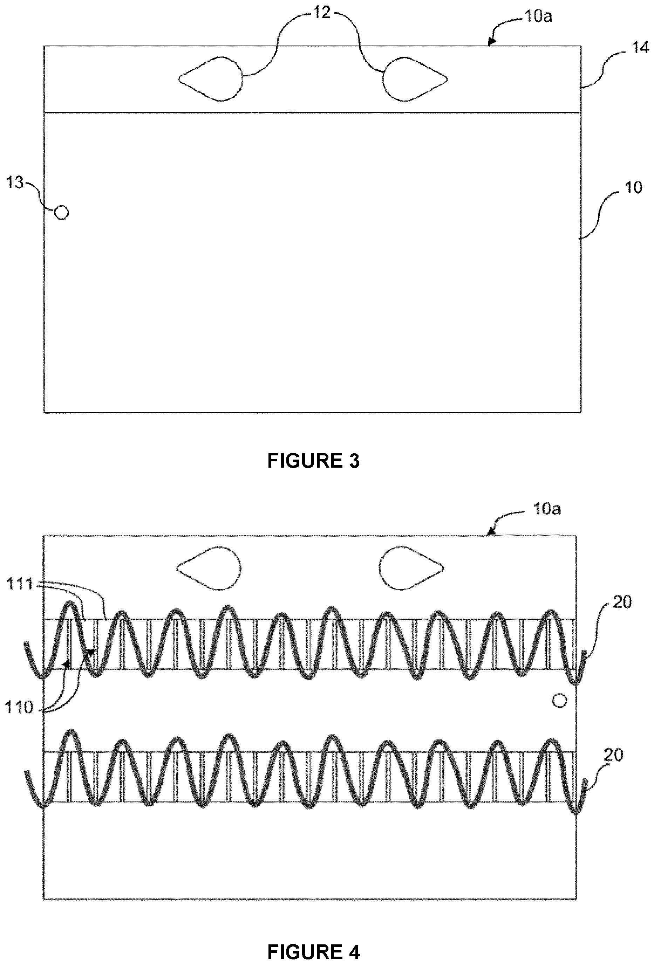

Les figures 2 et 3 présentent la bande de textile élastique destinée à former le tube, la figure 2 présentant la face intérieure (destinée à être en contact avec la peau de l'animal) et la figure 3 la face extérieure (opposée à la peau de l'animal).

La bande 10 présente une forme généralement rectangulaire. On désigne par 10a son bord antérieur (situé du côté des pattes avant de l'animal) et par 10b son bord postérieur (situé du côté des pattes arrière de l'animal).

De manière avantageuse, la bande est repliée sur elle-même au niveau du bord antérieur afin de former un rabat 14 qui est assemblé sur la face extérieure de la bande (cf. figure 2). Ce rabat permet de renforcer la bande, notamment lorsque des orifices y sont percés pour le passage des pattes.

Deux orifices 12 sont formés dans la partie avant de la bande, de préférence au travers du rabat 14 si celui-ci est présent. Ces orifices sont par exemple découpés au moyen d'un poinçon. De manière particulièrement avantageuse, les orifices 12 présentent une forme allongée dans le sens de la circonférence. Dans le mode de réalisation des figures 2 et 3, chaque ouverture 12 présente la forme d'une goutte d'eau, avec une portion semi-circulaire du côté intérieur de chaque patte et une portion effilée du côté extérieur de chaque patte.

Deux lanières rectangulaires 11 sont assemblées sur la face intérieure de la bande 10 selon des lignes 110 d'assemblage. Lesdites lanières sont perpendiculaires à l'axe longitudinal X de la bande, qui est parallèle à la colonne vertébrale de l'animal. Les lignes 110 sont parallèles à l'axe X. La distance d entre deux lignes 110 adjacentes est constante sur la largeur de la bande (correspondant à la circonférence du tube). Chaque paire de lignes adjacentes définit un passant respectif 111.

Un ou plusieurs orifices 13, qui peuvent être circulaires, sont ménagés au voisinage d'un bord latéral de la bande 10, afin de permettre le passage des fils électriquement conducteurs au travers du tube. Lesdits orifices sont par exemple découpés au moyen d'un poinçon.

La figure 4 représente la bande après enfilage d'un brin 20 de fil électriquement conducteur dans les passants de chaque lanière respective.

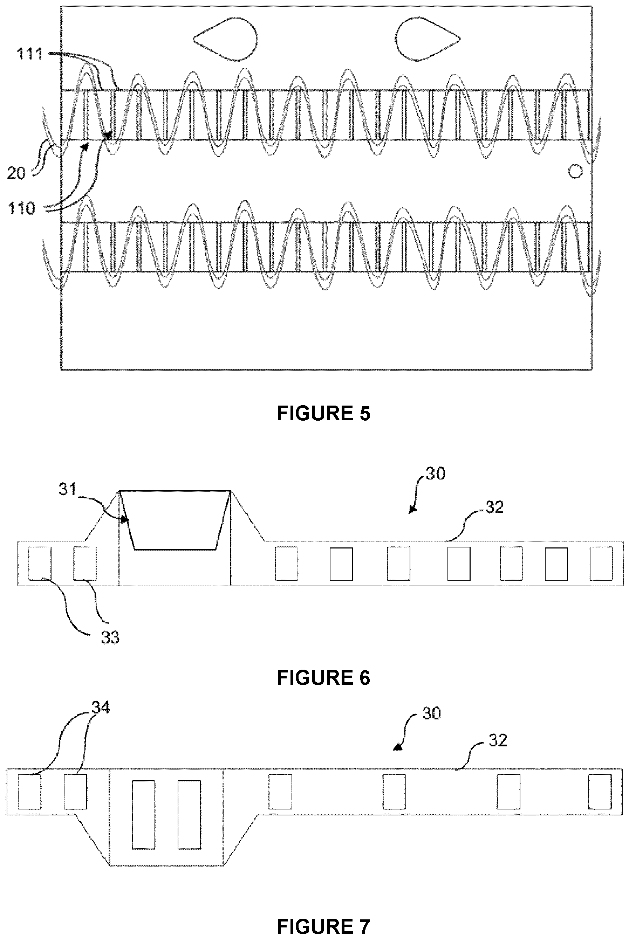

La figure 5 représente un mode de réalisation dans lequel deux brins 20 d'un même fil électriquement conducteur ont été enfilés dans les passants de chaque lanière respective pour former, après fermeture du tube, une bobine à deux spires.

Les figures 6 et 7 présentent respectivement la face extérieure et la face intérieure d'un mode de réalisation du harnais. Le harnais 30 comprend une poche 31 qui est formée par exemple par un rabat du textile extensible formant le harnais. Les dimensions intérieures de la poche sont adaptées à celle du connecteur susmentionné. De part et d'autre de ladite poche 31 s'étend une sangle 32 dont la longueur est adaptée pour entourer le tronc de l'animal. La face extérieure et la face intérieure de la sangle 32 comprennent des pièces d'attache complémentaires 33, 34, par exemple des rectangles de VELCRO™ répartis sur la longueur de la sangle. Ainsi, quelle que soit la section du tronc de l'animal, il est possible de mettre en regard au moins deux pièces complémentaires 33, 34 et ainsi ajuster la sangle au tronc de l'animal. La poche et la sangle ne sont pas nécessairement réalisées d'un seul tenant.

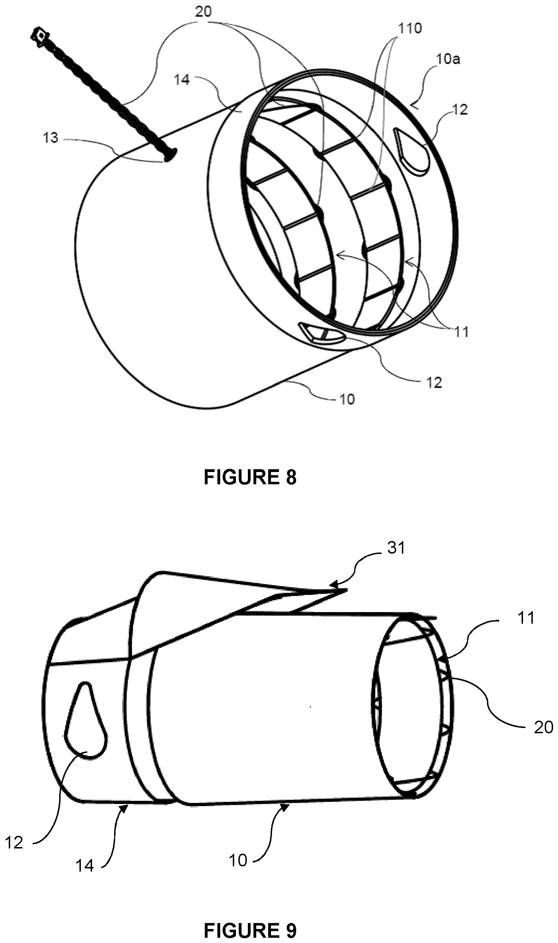

La figure 8 représente le gilet après fermeture de la bande sur elle-même pour former un tube. Le gilet comprend deux lanières 11 parallèles, maintenant deux spires électriquement conductrices formées chacune d'un brin 20 de fil électriquement conducteur. Les spires étant formées du côté intérieur du tube, les extrémités des brins de fil électriquement conducteur débouchent du tube par l'orifice 13 et sont raccordés à un connecteur. Les brins sont avantageusement torsadés les uns avec les autres.

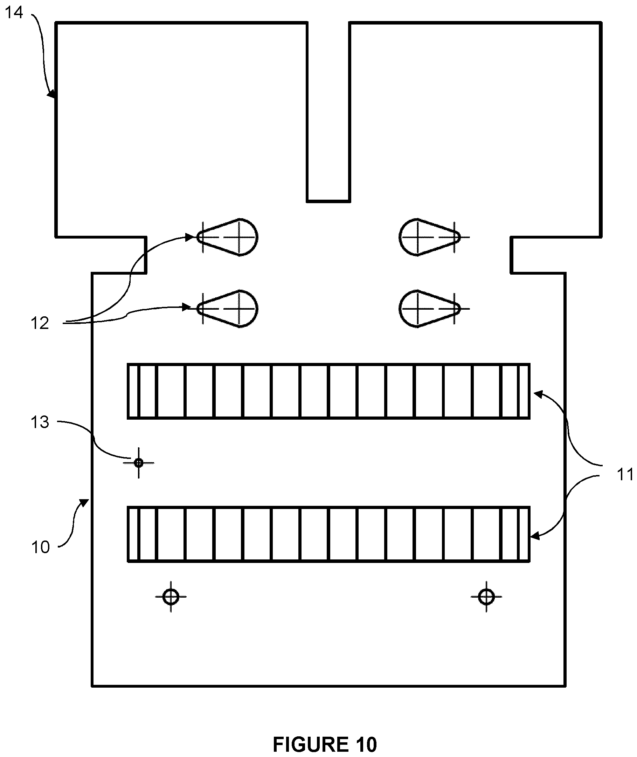

Les figures 9 et 10 illustrent un autre mode de réalisation du gilet, dans lequel la poche est intégrée au gilet, soit d'un seul tenant avec le gilet, soit par solidarisation directe avec le gilet. Ce mode de réalisation est avantageux en ce qu'il s'affranchit de la sangle, ce qui permet d'éviter d'exercer une compression trop importante de l'abdomen de l'animal. Par ailleurs, l'intégration de la poche au gilet permet d'éviter tout glissement de la poche autour du tronc de l'animal et d'assurer un bon centrage du dispositif d'acquisition par rapport au dos de l'animal.

Dans certains modes de réalisation, la poche est formée en assemblant deux languettes 14 parallèles formées d'un seul tenant avec la bande 10, au niveau de la partie destinée à former le col du gilet.

Lors de la fabrication du gilet, les languettes sont repliées sur elles-mêmes vers l'arrière de l'animal, et assemblées par tout moyen adapté (par exemple : collage, soudure, couture, etc.) pour former une poche de taille adaptée au dispositif d'acquisition. L'ouverture de la poche est avantageusement située au niveau du col, de sorte à être inaccessible lorsque la poche est rabattue sur le dos de l'animal.

Dans d'autres modes de réalisation, la poche est solidarisée au tube par tout moyen adapté (par exemple : collage, soudure, couture, etc.), de préférence au niveau de l'extrémité formant le col.

Par ailleurs, la bande 10 peut être rallongée vers l'arrière afin de former un rabat replié sur le dos de l'animal afin de recouvrir les câbles et électrodes du dispositif d'acquisition. Ainsi, le dispositif est protégé des projections de fluides ou d'attaques mécaniques notamment lorsque l'animal est en présence de congénères.

Applications

Une application du dispositif décrit ci-dessus est la pléthysmographie cardiaque par inductance pour mettre par exemple en oeuvre la bague de débit aortique virtuelle faisant l'objet du document WO 2017/037369 selon différentes configurations de mesure incluant, de manière non limitative :

- une bande thoracique, abdominale ou diaphragmatique,
- deux bandes thoraciques,
- deux bandes abdominales,
- une bande thoracique et une bande diaphragmatique,
- une bande diaphragmatique et une bande abdominale.

Une autre application du dispositif est la pléthysmographie respiratoire par inductance avec un modèle à un ou plusieurs compartiments dont les variations de section peuvent être enregistrées par :

- une bande thoracique ou abdominale,
- une bande abdominale et une bande thoracique,
- une bande thoracique, une bande abdominale et une bande diaphragmatique.

D'une manière générale, le dispositif permet le suivi des déformations mécaniques de compartiments avec autant de bandes thoraciques, diaphragmatiques ou abdominales que de compartiments à suivre.

Le gilet est à décliner selon différents modèles adaptés en fonction de l'espèce et du poids des animaux, qui est généralement compris entre 20 et 6000 g, de préférence entre 100 et 6000 g. Parmi les espèces pertinentes on recense notamment la souris, le rat, le cochon d'inde, le lapin, et des animaux juvéniles tels que le miniporc ou le chiot.

Par exemple pour une application chez des rats des souches Wistar ou Sprague Dawley, la gamme peut se composer de 4 tailles de gilets correspondants aux poids suivants :

|  | Gamme de poids |
|---|---|
| Taille 0 | 100 à 200g |
| Taille 1 | 200 à 300g |
| Taille 2 | 300 à 400g |
| Taille 3 | Au-delà de 400g |

Les positions abdominales, thoraciques et diaphragmatiques sont dépendantes de la morphologie de l'espèce et doivent être adaptées en conséquence.

REFERENCES

WO 2017/037369
US6341504
US7762953

**Revendications**

1. Gilet de pléthysmographie par inductance pour un mammifère présentant une masse inférieure à 6 kg, comprenant :

   - un tube (10) en textile élastique configuré pour entourer le tronc dudit mammifère ; ledit tube (10) s'étendant selon un axe longitudinal (X) adapté pour être parallèle à la colonne vertébrale dudit mammifère ;
   - au moins une spire inductive formée d'un brin (20) de fil électriquement conducteur agencé sous forme de vaguelettes selon la circonférence dudit tube (10) ;

   ledit gilet étant **caractérisé en ce qu'**il comprend au moins une lanière (11) en textile élastique, agencée le long de la circonférence du tube, définissant une pluralité de passants (111) longitudinaux, chaque vaguelette étant formée par le passage du brin (20) de fil conducteur successivement dans chaque passant (111).

2. Gilet selon la revendication 1, dans lequel chaque passant est délimité par deux lignes longitudinales (110) d'assemblage de la lanière (11) sur le tube (10).

3. Gilet selon la revendication 2, dans lequel la distance (d) entre deux lignes (110) d'assemblage adjacentes est constante.

4. Gilet selon l'une des revendications 1 à 3, dans lequel le tube comprend en outre deux orifices (12) adaptés pour le passage des pattes avant du mammifère, chaque orifice présentant une forme allongée selon la circonférence du tube.

5. Gilet selon la revendication 4, dans lequel le tube comprend en outre un renfort formé par un rabat (14) du tube du côté des pattes avant du mammifère, lesdits orifices (12) s'étendant au travers dudit rabat.

6. Gilet selon l'une des revendications 1 à 5, comprenant en outre un logement (31) pour un dispositif d'acquisition connecté à chaque spire inductive, ledit logement étant formé d'un seul tenant avec le tube (10) ou solidarisé directement au tube.

**7.** Gilet selon l'une des revendications 1 à 5, comprenant en outre un harnais (30) comprenant un logement (31) pour un dispositif d'acquisition connecté à chaque spire inductive et une sangle (32) adaptée pour maintenir le tube (10) autour de l'abdomen du mammifère.

**8.** Gilet selon la revendication 7, dans lequel la sangle (32) est pourvue d'une pluralité de pièces agrippantes (33) de type crochets et boucles.

**9.** Gilet selon l'une des revendications 1 à 8, dans lequel chaque brin (20) de fil électriquement conducteur passe au moins deux fois dans chaque passant (111) selon la circonférence du tube, de sorte à former un ensemble de spires constituant une bobine.

**10.** Procédé de fabrication d'un gilet de pléthysmographie par inductance pour un mammifère de masse inférieure à 6 kg, comprenant :

- la fourniture d'une bande (10) en textile élastique présentant un axe longitudinal (X) ;
- l'assemblage sur la bande (10) d'au moins une lanière (11) en textile élastique perpendiculaire à l'axe longitudinal, selon une pluralité de lignes (110) parallèles audit axe longitudinal, de sorte à définir une pluralité de passants (111) entre deux lignes (110) adjacentes de ladite lanière ;
- la fermeture de la bande (10) selon une ligne parallèle à l'axe longitudinal (X) de sorte à former un tube ;
- la formation d'au moins une spire inductive selon la circonférence du tube en passant un brin (20) de fil électriquement conducteur successivement dans chaque passant (111) d'une lanière respective de sorte à former des vaguelettes.

**11.** Procédé selon la revendication 10, dans lequel l'assemblage de la lanière (11) sur le tube est réalisé par soudure par ultrason, couture et/ou collage.

**12.** Procédé selon l'une des revendications 10 ou 11, comprenant en outre le perçage dans la bande (10) de deux orifices (12) adaptés pour le passage des pattes avant du petit mammifère au moyen d'un poinçon.

**13.** Procédé selon la revendication 12, comprenant en outre, avant le perçage desdits orifices (12), l'assemblage d'un rabat (14) de la bande (10) sur une face du tube, les orifices (12) étant percés au travers dudit rabat (14).

**14.** Procédé de fabrication d'un ensemble de gilets de pléthysmographie par inductance pour des petits mammifères de tailles différentes, la masse desdits mammifères étant comprise entre 20 et 6000 g, dans lequel on met en oeuvre le procédé selon l'une des revendications 10 à 13 pour chacun desdits gilets, la distance (d) entre deux lignes (110) d'assemblage adjacentes de chaque lanière étant identique pour l'ensemble des gilets.

**Patentansprüche**

**1.** Weste zur induktiven Plethysmographie für ein Säugetier mit einer Masse von weniger als 6 kg, umfassend:

- einen Schlauch (10) aus elastischem Textilmaterial, der dazu konfiguriert ist, den Oberkörper des Säugetiers zu umgeben; wobei sich der Schlauch (10) entlang einer Längsachse (X) erstreckt, die dazu ausgelegt ist, parallel zur Wirbelsäule des Säugetiers zu verlaufen;
- mindestens eine induktive Wicklung, die aus einem Strang (20) aus elektrisch leitendem Draht gebildet wird, der wellenförmig entlang des Umfangs des Schlauchs (10) angeordnet ist;

wobei die Weste **dadurch gekennzeichnet ist, dass** sie mindestens einen Riemen (11) aus elastischem Textilmaterial umfasst, der entlang des Umfangs des Schlauchs angeordnet ist und eine Vielzahl von Längsschlaufen (111) definiert, wobei jede Welle durch die aufeinanderfolgenden Durchführung des Strangs (20) aus leitendem Draht in jeder Schlaufe (111) gebildet wird.

**2.** Weste nach Anspruch 1, wobei jede Schlaufe durch zwei längliche Anbringungslinien (110) für den Riemen (11) mit dem Schlauch (10) begrenzt ist.

**3.** Weste nach Anspruch 2, wobei der Abstand (d) zwischen zwei benachbarten Anbringungslinien (110) konstant ist.

**4.** Weste nach einem der Ansprüche 1 bis 3, wobei der Schlauch ferner zwei Öffnungen (12) umfasst, die für die Durchführung der Vorderbeine des Säugetiers ausgelegt sind, wobei jede Öffnung entlang des Umfangs des Schlauchs eine längliche Form aufweist.

**5.** Weste nach Anspruch 4, wobei der Schlauch ferner eine Verstärkung umfasst, die durch einen Umschlag (14) des Schlauchs auf der Seite der Vorderbeine des Säugetiers gebildet wird, wobei sich die Öffnungen (12) durch den Umschlag erstrecken.

**6.** Weste nach einem der Ansprüche 1 bis 5, die ferner eine Aufnahme (31) für eine Erfassungsvorrichtung umfasst, die mit jeder Induktionswicklung verbunden ist, wobei die Aufnahme einstückig mit dem Schlauch (10) ausgebildet oder direkt integral mit dem Schlauch hergestellt ist.

7. Weste nach einem der Ansprüche 1 bis 5, die ferner einen Gurt (30) umfasst, der eine Aufnahme (31) für eine Erfassungsvorrichtung, die mit jeder Induktionswicklung verbunden ist, und ein Gurtband (32) umfasst, das dazu ausgelegt ist, den Schlauch (10) um den Bauch des Säugetiers zu halten.

8. Weste nach Anspruch 7, wobei das Gurtband (32) mit einer Vielzahl von Greifverbindungsteilen (33) vom Klettverschlusstyp versehen ist.

9. Weste nach einem der Ansprüche 1 bis 8, wobei jeder Strang (20) aus elektrisch leitendem Draht mindestens zweimal in jeder Schlaufe (111) entlang des Umfangs des Schlauchs verläuft, um einen Satz von Windungen zu bilden, die eine Spule darstellen.

10. Verfahren zum Herstellen einer Weste zur induktiven Plethysmographie für ein Säugetier mit einer Masse von weniger als 6 kg, umfassend:

    - Bereitstellen eines Bands (10) aus elastischem Textilmaterial, das eine Längsachse (X) aufweist;
    - Anbringen mindestens eines Riemens (11) aus elastischem Textilmaterial senkrecht zur Längsachse (X) entlang einer Vielzahl von Linien (110), die parallel zur Längsachse verlaufen, an dem Band (10), um eine Vielzahl von Schlaufen (111) zwischen zwei benachbarten Linien (110) des Riemens zu definieren;
    - Schließen des Bands (10) entlang einer Linie, die parallel zur Längsachse (X) verläuft, um einen Schlauch zu bilden;
    - Bilden mindestens einer induktiven Wicklung entlang eines Umfangs des Schlauchs durch das Durchführen eines Strangs (20) aus elektrisch leitendem Draht nacheinander in jeder Schlaufe (111) eines jeweiligen Riemens, um Wellen zu bilden.

11. Verfahren nach Anspruch 10, wobei das Anbringen des Riemens (11) an dem Schlauch durch Ultraschallschweißen, Nähen und/oder Kleben durchgeführt wird.

12. Verfahren nach einem der Ansprüche 10 oder 11, das ferner das Durchstechen von zwei Öffnungen (12), die für die Durchführung der Vorderbeine des kleinen Säugetiers ausgelegt sind, in das Band (10) mit Hilfe einer Stanze umfasst.

13. Verfahren nach Anspruch 12, das ferner vor dem Durchstechen der Öffnungen (12) das Anbringen einer Umschlags (14) des Bands (10) auf einer Seite des Schlauchs umfasst, wobei die Öffnungen (12) durch den Umschlag (14) gestochen werden.

14. Verfahren zum Herstellen eines Satzes Westen zur induktiven Plethysmographie für kleine Säugetiere unterschiedlicher Größe, wobei die Masse der Säugetiere zwischen 20 und 6000 g beträgt, wobei das Verfahren nach einem der Ansprüche 10 bis 13 für jede der Westen durchgeführt wird, wobei der Abstand (d) zwischen zwei benachbarten Anbringungslinien (110) jedes Riemens identisch für den Satz Westen ist.

## Claims

1. Inductance plethysmography vest for a mammal with a mass of less than 6 kg, comprising:

    - a tube (10) of elastic textile configured to surround the trunk of said mammal; said tube (10) extending along a longitudinal axis (X) adapted to be parallel to the spine of said mammal;
    - at least one inductive turn formed by a strand (20) of electrically conductive wire arranged in the form of wavelets around the circumference of said tube (10);

    said vest being **characterized in that** it comprises at least one elastic textile strip (11), arranged along the circumference of the tube, defining a plurality of longitudinal loops (111), each wavelet being formed by the passage of the strand (20) of conductive thread successively through each loop (111).

2. Vest according to claim 1, wherein each loop is delimited by two longitudinal lines (110) for joining the strip (11) to the tube (10).

3. Vest according to claim 2, wherein the distance (d) between two adjacent assembly lines (110) is constant.

4. Vest according to one of claims 1 to 3, wherein the tube further comprises two holes (12) adapted for the passage of the mammal's front legs, each hole having an elongated shape along the circumference of the tube.

5. Vest according to claim 4, wherein the tube further comprises a reinforcement formed by a flap (14) of the tube on the side of the mammal's front legs, said holes (12) extending through said flap.

6. Vest according to one of claims 1 to 5, further comprising a housing (31) for an acquisition device connected to each inductive turn, said housing being formed integrally with the tube (10) or attached directly to the tube.

7. Vest according to one of claims 1 to 5, further comprising a harness (30) including a housing (31) for an acquisition device connected to each inductive turn and a strap (32) adapted to hold the tube (10) around the mammal's abdomen.

8. Vest according to claim 7, wherein the strap (32) is provided with a plurality of hook-and-loop fasteners (33).

9. Vest according to one of claims 1 to 8, wherein each strand (20) of electrically conductive wire passes at least twice through each loop (111) along the circumference of the tube, so as to form a set of turns constituting a coil.

10. A method of manufacturing an inductance plethysmography vest for a mammal of mass less than 6 kg, comprising:

> - the provision of an elastic textile band (10) with a longitudinal axis (X);
> - assembling on the band (10) at least one elastic textile strip (11) perpendicular to the longitudinal axis, along a plurality of lines (110) parallel to said longitudinal axis, so as to define a plurality of loops (111) between two adjacent lines (110) of said strip;
> - closing the band (10) along a line parallel to the longitudinal axis (X) to form a tube;
> - forming at least one inductive turn around the circumference of the tube by passing a strand (20) of electrically conductive wire successively through each loop (111) of a respective strip so as to form wavelets.

11. Method according to claim 10, in which the strip (11) is assembled to the tube by ultrasonic welding, sewing and/or gluing.

12. Process according to one of claims 10 or 11, further comprising the drilling in the band (10) of two holes (12) adapted for the passage of the front legs of the small mammal by means of a punch.

13. Method according to claim 12, further comprising, prior to drilling said holes (12), assembling a flap (14) of the band (10) on one face of the tube, the holes (12) being drilled through said flap (14).

14. Process for manufacturing a set of inductance plethysmography vests for small mammals of different sizes, the mass of said mammals being between 20 and 6000 g, in which the process according to one of claims 10 to 13 is carried out for each of said vests, the distance (d) between two adjacent assembly lines (110) of each strip being identical for all the vests.

**FIGURE 1**

**FIGURE 2**

**FIGURE 3**

**FIGURE 4**

**FIGURE 5**

**FIGURE 6**

**FIGURE 7**

**FIGURE 8**

**FIGURE 9**

**FIGURE 10**

**RÉFÉRENCES CITÉES DANS LA DESCRIPTION**

**Documents brevets cités dans la description**

- US 6341504 B
- US 7762953 B
- US 5543012 A
- US 20180317814 A
- WO 2017037369 A
- EP 1404219 A